# EUROPEAN PATENT APPLICATION

(11) **EP 4 316 348 A1**
(43) Date of publication of application: **07.02.2024**
(21) Application number: 21859355.6
(22) Date of filing: 29.03.2021
(51) Int. Cl.: A61B 1/045

(54) **IMAGE PROCESSING DEVICE, IMAGE PROCESSING METHOD, AND STORAGE MEDIUM**

(71) Applicant: NEC Corporation, Tokyo 108-8001 (JP)
(72) Inventor: SHINO, Ryosaku, Tokyo 108-8001 (JP)
(74) Representative: Betten & Resch
(86) International application number: PCT/JP2021/013314
(87) International publication number: WO 2022/208615

(57) **Abstract**

The image processing device 1X mainly includes an acquisition means 30X, a lesion part detection means 31X, and a lesion diagnosis means 32X. The acquisition means 30X acquires a captured image acquired by photographing an inspection target by a photographing unit provided in an endoscope. The lesion part detection means 31X detects a lesion part, in which a lesion formation is suspected, in the captured image. The lesion diagnosis means 32X makes a diagnosis relating to the lesion part based on the captured image and a cutout image which indicates the lesion part cut out from the captured image.

## Description

### TECHNICAL FIELD

The present disclosure relates to a technical field of an image processing device, an image processing method, and storage medium for processing images acquired in endoscopic inspection.

### BACKGROUND ART

An endoscopic system for displaying images taken in the lumen of an organ is known. For example, Patent Literature 1 discloses an endoscopic inspection system which detects a lesion part based on the hue, the size, and the like of an image taken by an endoscope to thereby display and highlight the detected lesion part.

### CITATION LIST

### PATENT LITERATURE

Patent Literature 1: JP 2011-135983A

### SUMMARY

### PROBLEM TO BE SOLVED BY THE INVENTION

According to Patent Literature 1, after detecting a lesion part, the lesion part is highlighted, but it has been left to the inspector to specifically diagnose the state of the detected lesion part. On the other hand, in some cases, diagnosis by the inspector is difficult depending on the lesion.

In view of the above-described issue, it is therefore an example object of the present disclosure to provide an image processing device, an image processing method, and a storage medium capable of suitably making a diagnosis of a lesion part in endoscopic inspection.

### MEANS FOR SOLVING THE PROBLEM

One mode of the image processing device is an image processing device including:
an acquisition means for acquiring a captured image acquired by photographing an inspection target by a photographing unit provided in an endoscope;
a lesion part detection means for detecting a lesion part, in which a lesion formation is suspected, in the captured image; and
a lesion diagnosis means for making a diagnosis relating to the lesion part based on the captured image and a cutout image which indicates the lesion part cut out from the captured image.

One mode of the image processing method is an image processing method executed by a computer, the image processing method including:
acquiring a captured image acquired by photographing an inspection target by a photographing unit provided in an endoscope;
detecting a lesion part, in which a lesion formation is suspected, in the captured image; and
making a diagnosis relating to the lesion part based on the captured image and a cutout image which indicates the lesion part cut out from the captured image.

One mode of the storage medium is a storage medium storing a program executed by a computer, the program causing the computer to:
acquire a captured image acquired by photographing an inspection target by a photographing unit provided in an endoscope;
detect a lesion part, in which a lesion formation is suspected, in the captured image; and
make a diagnosis relating to the lesion part based on the captured image and a cutout image which indicates the lesion part cut out from the captured image.

### EFFECT OF THE INVENTION

An example advantage according to the present invention is to suitably make a diagnosis of a lesion part from an image of an endoscope.

### BRIEF DESCRIPTION OF THE DRAWINGS

FIG. 1 illustrates a schematic configuration of an endoscopic inspection system.
FIG. 2 illustrates a hardware configuration of an image processing device.
FIG. 3 illustrates an outline of a process performed by the image processing device.
FIG. 4 is a functional block diagram of the image processing device.
FIG. 5A illustrates a first example of the generation of an input image to be inputted to a lesion diagnosis model.
FIG. 5B illustrates a second example of the generation of the input image to be inputted to the lesion diagnosis model.
FIG. 6 illustrates a display example of the display view displayed by the display device in endoscopic inspection.
FIG. 7 is an example of a flowchart showing an outline of the process executed by the image processing device according to the first example embodiment.
FIG. 8 is a schematic configuration diagram of an endoscopic inspection system in a modification.
FIG. 9 is a block diagram of an image processing device according to a second example embodiment.
FIG. 10 is an example of a flowchart to be executed by the image processing device according to the second example embodiment.

### EXAMPLE EMBODIMENTS

Hereinafter, an example embodiment of an image processing device, an image processing method, and a storage medium will be described with reference to the drawings.

### <First Example Embodiment>

### (1) System Configuration

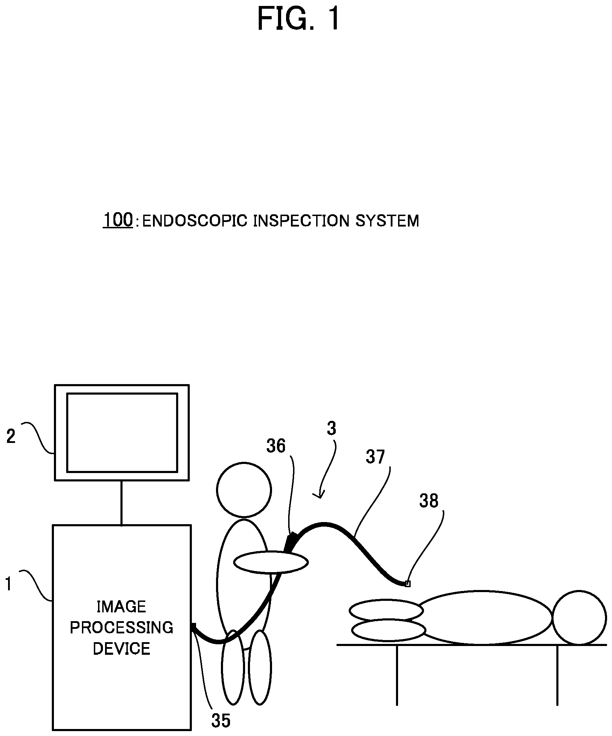

FIG. 1 shows a schematic configuration of an endoscopic inspection system 100. The endoscopic inspection system 100 detects a part (a lesion part), in which a lesion formation is suspected, for an inspector such as a doctor who performs an inspection (including treatment) using an endoscope, and makes a diagnosis of the detected lesion part (diseased part). As shown in FIG. 1, the endoscopic inspection system 100 mainly includes an image processing device 1, a display device 2, and an endoscope 3 connected to the image processing device 1.

The image processing device 1 acquires an image (also referred to as "captured image Ia") taken by the endoscope 3 in time series from the endoscope 3 and displays a view based on the captured image Ia on the display device 2. The captured image Ia is an image taken at predetermined time intervals during at least one of the insertion process of the endoscope 3 to the subject or the ejection process of the endoscope 3. In the present example embodiment, the image processing device 1 analyzes the captured image Ia and thereby performs detection of a lesion part and the diagnosis of the detected lesion part to display information on the diagnosis result on the display device 2.

The display device 2 is a display or the like for displaying information based on a display signal supplied from the image processing device 1.

The endoscope 3 mainly includes an operation unit 36 for inspector to perform a predetermined input, a shaft 37 which has flexibility and which is inserted into the organ to be photographed of the subject, a pointed end unit 38 having a built-in photographing unit such as an ultra-small image pickup device, and a connecting unit 39 for connecting with the image processing device 1.

In the following description, as a representative example, the process in the endoscopic inspection of the large bowel will be explained, but the inspection target is not limited to the large bowel, and the inspection target may be a digestive canal (digestive organ) such as a stomach, an esophagus, a small bowel, and a duodenum. It is noted that the image processing device 1 according to the present example embodiment using the method described later can accurately make the diagnosis of identifying the lesion (lesions) such as a hyperplastic polyp, a serrated lesion, the adenoma, and the carcinoma in situ, which are difficult to diagnose without considering the difference between the surface patterns of the normal area and the lesion part. The above-mentioned lesion can be formed not only in the large bowel but also in the stomach. Thus, the endoscopic inspection system 100 can precisely make a diagnosis that identify the lesion as described above and suitably assist the inspector, at least when inspecting the large bowel and the stomach. It is also noted that the image processing device 1 can make a diagnosis not only for identifying such a lesion difficult to diagnose without considering the difference between the surface patterns of the normal area and the lesion part but also for identifying any other lesions.

### (2) Hardware Configuration

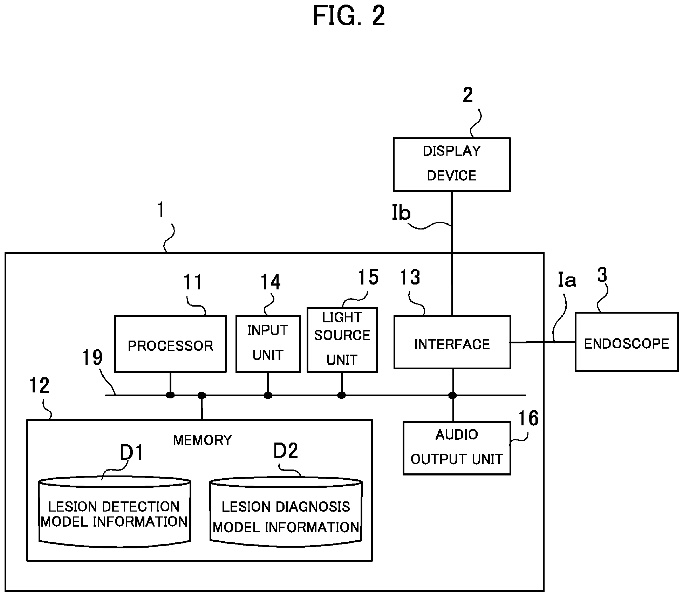

FIG. 2 shows the hardware configuration of the image processing device 1. The image processing device 1 mainly includes a processor 11, a memory 12, an interface 13, an input unit 14, a light source unit 15, and an audio output unit 16. Each of these elements is connected via a data bus 19.

The processor 11 executes a predetermined process by executing a program or the like stored in the memory 12. The processor 11 is one or more processors such as a CPU (Central Processing Unit), a GPU (Graphics Processing Unit), and a TPU (Tensor Processing Unit). The processor 11 may be a plurality of processors. The processor 11 is an example of a computer.

The memory 12 is configured by a variety of volatile memories used as working memories, and nonvolatile memories that store information necessary for the process to be executed by the image processing device 1, such as a RAM (Random Access Memory) and a ROM (Read Only Memory). The memory 12 may include an external storage device such as a hard disk connected to or built in to the image processing device 1, or may include a storage medium such as a removable flash memory. The memory 12 stores a program for the image processing device 1 to execute each processing in the present example embodiment.

The memory 12 stores the lesion detection model information D1 and the lesion diagnosis model information D2.

The lesion detection model information D1 is information on a lesion detection model, which is a model for detecting a lesion part from an input image. The lesion detection model information D1 includes parameters necessary to configure the lesion detection model. The lesion detection model is, for example, a machine learning model such as a neural network or a support vector machine, and is a model configured to output a detection result of a lesion part (a part having an abnormality) in the inputted captured image Ia when the captured image Ia is inputted. Examples of a typical model of a neural network to be used for such a lesion detection model include Fully Convolutional Network, SegNet, U-Net, V-Net, Feature Pyramid Network, Mask R-CNN, and DeepLab. The detection result outputted by the lesion detection model may be a mask image (a binary image in which the values are different between the pixels of the lesion part and the other pixels) indicating an area in the captured image Ia corresponding to the lesion part, or may be a reliability map that is a map on the image indicating the reliability of being the lesion part. Here, the reliability is an index representing the degree of the possibility of being a lesion part, which indicates that the higher the reliability is, the higher the possibility of being a lesion part is. In yet another example, the lesion detection model may be a model configured to output information indicative of a rectangular area (bounding box) of the lesion part on the inputted captured image Ia when the captured image Ia is inputted. The image processing device 1 generates a (cropped) image (also referred to as a " cutout lesion image Ic") that is an area including a lesion part cut out from the captured image Ia based on the detection result outputted by the lesion detection model.

The lesion diagnosis model information D2 is information on the lesion diagnosis model which is a model configured to diagnose the lesion part shown in an input image inputted thereto. The lesion diagnosis model information D2 includes parameters necessary to configure the lesion diagnosis model. The lesion diagnosis model is, for example, a machine learning model such as a neural network or a support vector machine, and is a model configured to output a diagnosis result of the lesion part indicated by the cutout lesion image Ic when the cutout lesion image Ic and the corresponding captured image Ia are simultaneously inputted (i.e., inputted as one input image) to the model. The diagnosis result outputted by the lesion diagnosis model is, for example, classification information indicating the type (category) of the lesion or the lesion name corresponding to the target lesion part. This classification information may include a classification result regarding the risk of the lesion, such as the possibility of the cancerization of the target lesion part.

The lesion detection model and the lesion diagnosis model are trained in advance based on plural sets of input images and corresponding correct answer data, wherein each of the input images conforms to the input format of each model and the correct answer data indicates the correct answer to be outputted when the input image is inputted to each model. Then, parameters and the like of each model acquired by training are stored in the memory 12 as the lesion detection model information D1 and the lesion diagnosis model information D2, respectively.

At least one of the lesion detection model information D1 or the lesion diagnosis model information D2 may be stored in an external device capable of data communication with the image processing device 1 by wired or wirelessly, instead of being stored in the memory 12.

The interface 13 performs an interface operation between the image processing device 1 and an external device. For example, the interface 13 supplies the display information "Ib" generated by the processor 11 to the display device 2. Further, the interface 13 supplies the light generated by the light source unit 15 to the endoscope 3. The interface 13 also provides the processor 11 with an electrical signal indicative of the captured image Ia supplied from the endoscope 3. The interface 13 may be a communication interface, such as a network adapter, for wired or wireless communication with an external device, or a hardware interface conforming to a USB (Universal Serial Bus), a SATA (Serial AT Attachment), or the like.

The input unit 14 generates an input signal based on the operation by the inspector. Examples of the input unit 14 include a button, a touch panel, a remote controller, and a voice input device. The light source unit 15 generates light for supplying to the pointed end unit 38 of the endoscope 3. The light source unit 15 may also incorporate a pump or the like for delivering water and air to be supplied to the endoscope 3. The audio output unit 16 outputs a sound under the control of the processor 11.

### (3) Process by Image Processing Device

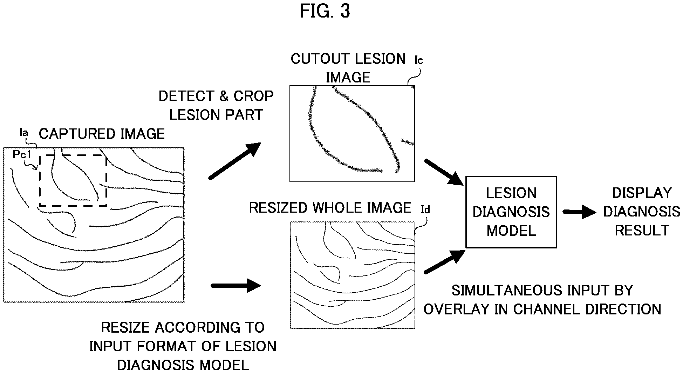

FIG. 3 illustrates an outline of the process performed by the image processing device 1.

First, the image processing device 1 generate a cutout lesion image Ic by cutting out (cropping) an area corresponding to the lesion part from the captured image Ia based on the lesion detection model configured by the lesion detection model information D1. Further, the image processing device 1 generates an image (also referred to as "resized whole image Id") in which the captured image Ia used for cutting out the cutout lesion image Ic is resized according to the input format of the lesion diagnosis model. In this case, the image processing device 1 performs a process of converting the captured image Ia into the resized whole image Id using an arbitrary resizing technique of an image.

Next, when a set of the cutout lesion image Ic and the resized whole image Id is acquired, the image processing device 1 simultaneously inputs the cutout lesion image Ic and the resized whole image Id to the lesion diagnosis model configured by the lesion diagnosis model information D2, and therefore acquires from the model the diagnosis result for the lesion part indicated by the cutout lesion image Ic. In this case, by overlaying the cutout lesion image Ic on the resized whole image Id in the channel direction, the image processing device 1 generates an input image, into which the cutout lesion image Ic and the resized whole image Id are combined, to the lesion diagnosis model. Then, the image processing device 1 displays information on the diagnosis result on the display device 2.

By executing such a process, the image processing device 1 can accurately make the diagnosis to identify each lesion such as a hyperplastic polyp, a serrated lesion, the adenoma, and the carcinoma in situ, which are difficult to diagnose without considering the difference between the surface patterns of the normal area and the lesion part, and present the diagnosis result to the inspector.

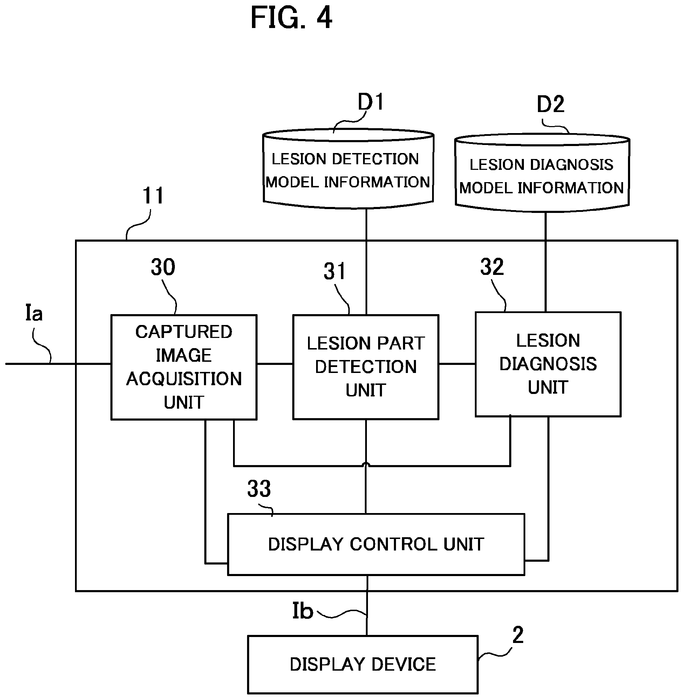

FIG. 4 is a functional block diagram of the image processing device 1. As shown in FIG. 4, the processor 11 of the image processing device 1 functionally includes a captured image acquisition unit 30, a lesion part detection unit 31, a lesion diagnosis unit 32, and a display control unit 33. In FIG. 4, each combination of the blocks to exchange data with each other are connected by solid line. However, the combinations of blocks to exchange data with each other are not limited as shown in FIG. 4. It is true for the other functional block diagrams to be described later.

The captured image acquisition unit 30 acquires the captured image Ia taken by the endoscope 3 via the interface 13 at predetermined intervals. Then, the captured image acquisition unit 30 supplies the acquired captured image Ia to the lesion part detection unit 31, the lesion diagnosis unit 32, and the display control unit 33, respectively.

The lesion part detection unit 31 detects the lesion part based on the captured image Ia. In this case, the lesion part detection unit 31 inputs the captured image Ia to the lesion detection model configured by the lesion detection model information D1, and acquires the detection result outputted by the lesion detection model. Then, when determining that there is a lesion part in the captured image Ia based on the acquired detection result, the lesion part detection unit 31 generates a cutout lesion image Ic based on the detection result, and supplies the cutout lesion image Ic to the lesion diagnosis unit 32.

Here, a supplementary description will be given of examples of the generation of the cutout lesion image Ic based on the detection result outputted by the lesion detection model. For example, when the detection result outputted by the lesion detection model indicates pixels of the lesion part, the lesion part detection unit 31 cuts out the smallest rectangular area including the pixels from the captured image Ia to thereby generate the cutout lesion image Ic. In another example, when the detection result outputted by the lesion detection model indicates the reliability of the lesion part for each pixel, the lesion part detection unit 31 cuts out, from the captured image Ia, the minimum rectangular area including pixels whose reliability is equal to or higher than a predetermined threshold value to thereby generate the cutout lesion image Ic. In yet another example, when the detection result outputted by the lesion detection model indicates a rectangular area (i.e., bounding box) of the lesion part, the lesion part detection unit 31 generates a cutout lesion image Ic by cutting out the rectangular area from the captured image Ia.

The lesion diagnosis unit 32 makes a diagnosis of the lesion part detected by the lesion part detection unit 31. In this case, first, the lesion diagnosis unit 32 acquires a cutout lesion image Ic supplied from the lesion part detection unit 31, and a captured image Ia serving as a cutting source (original image) of the cutout lesion image Ic. Next, the lesion diagnosis unit 32 generates the resized whole image Id acquired by resizing the acquired captured image Ia based on the input format of the lesion diagnosis model. Then, the lesion diagnosis unit 32 inputs an image, which is acquired by combining the cutout lesion image Ic and the resized whole image Id as one input image, to the lesion diagnosis model configured by the lesion diagnosis model information D2. Then, the lesion diagnosis unit 32 acquires the diagnosis result outputted by the lesion diagnosis model. In this way, the lesion diagnosis department 32 makes a diagnosis regarding the lesion part based on the diagnosis result outputted by the lesion diagnosis model. Furthermore, the lesion diagnosis unit 32 supplies the diagnosis result to the display control unit 33.

The lesion diagnosis unit 32 may supply the diagnosis result outputted by the lesion diagnosis model to the display control unit 33 as it is, or may supply the display control unit 33 with the diagnosis result enhanced based on the prior knowledge information on the lesion classification stored in the memory 12 or the like. The prior knowledge information includes, for example, at least one of information which relates the classification of lesions to the risk of each of the lesions such as cancerization risk, or information which relates the classification of lesions to the lesion name corresponding to each of the lesions. For example, when the information which relates the classification of the lesions and the risk of each of the lesions such as cancerization risk is stored in the memory 12 as the prior knowledge information, the lesion diagnosis unit 32 specifies the corresponding risk by referring to the prior knowledge information based on the classification of the lesion that the lesion diagnosis model outputs as the diagnosis result. Then, the lesion diagnosis unit 32 may supply the classification information of the lesion outputted by the lesion diagnosis model and the information on the risk of the lesion based on the prior knowledge information to the display control unit 33 as the diagnosis result. In addition, when the information which relates the classification of lesions to the lesion name corresponding to each of the lesions is stored in the memory 12 as the prior knowledge information, the lesion diagnosis unit 32 may supply the diagnosis result, which includes the lesion name according to the prior knowledge information, to the display control unit 33.

Here, each of the process of generating the resized whole image Id by resizing the captured image Ia based on the input format of the lesion diagnosis model, and the process of combining the cutout lesion image Ic and the resized whole image Id as one input image is not an essential process to be executed by the lesion diagnosis unit 32. At least one of these processes may be executed by a processing block other than the lesion diagnosis unit 32 (including a processing block not shown in FIG. 4). For example, when the other processing block executes the process of generating the resized whole image Id, the lesion diagnosis unit 32 acquires the resized whole image Id into which the captured image Ia is resized based on the input format of the lesion diagnosis model from the other processing block. In addition, when the other processing block executes the process of combining the cutout lesion image Ic and the resized whole image Id as one input image, the lesion diagnosis unit 32 acquires an image into which the cutout lesion image Ic and the resized whole image Id are combined as an input image of the lesion diagnosis model from the other processing block.

The display control unit 33 generates a display information Ib based on the captured image Ia, and the detection result of the lesion part by the lesion part detection unit 31, and the diagnosis result of the lesion part by the lesion diagnosis unit 32, and supplies the display information Ib via the interface 13 to the display device 2. Specific examples of the display control of the display device 2 by the display control unit 33 will be described later.

Here, for example, each component of the captured image acquisition unit 30, the lesion part detection unit 31, the lesion diagnosis unit 32 and the display control unit 33 can be realized by the processor 11 executing a program. In addition, the necessary program may be recorded in any non-volatile storage medium and installed as necessary to realize the respective components. In addition, at least a part of these components is not limited to being realized by a software program and may be realized by any combination of hardware, firmware, and software. At least some of these components may also be implemented using user-programmable integrated circuitry, such as FPGA (Field-Programmable Gate Array) and microcontrollers. In this case, the integrated circuit may be used to realize a program for configuring each of the above-described components. Further, at least a part of the components may be configured by a ASSP (Application Specific Standard Produce), ASIC (Application Specific Integrated Circuit) and/or a quantum processor (quantum computer control chip). In this way, each component may be implemented by a variety of hardware. The above is true for other example embodiments to be described later. Further, each of these components may be realized by the collaboration of a plurality of computers, for example, using cloud computing technology.

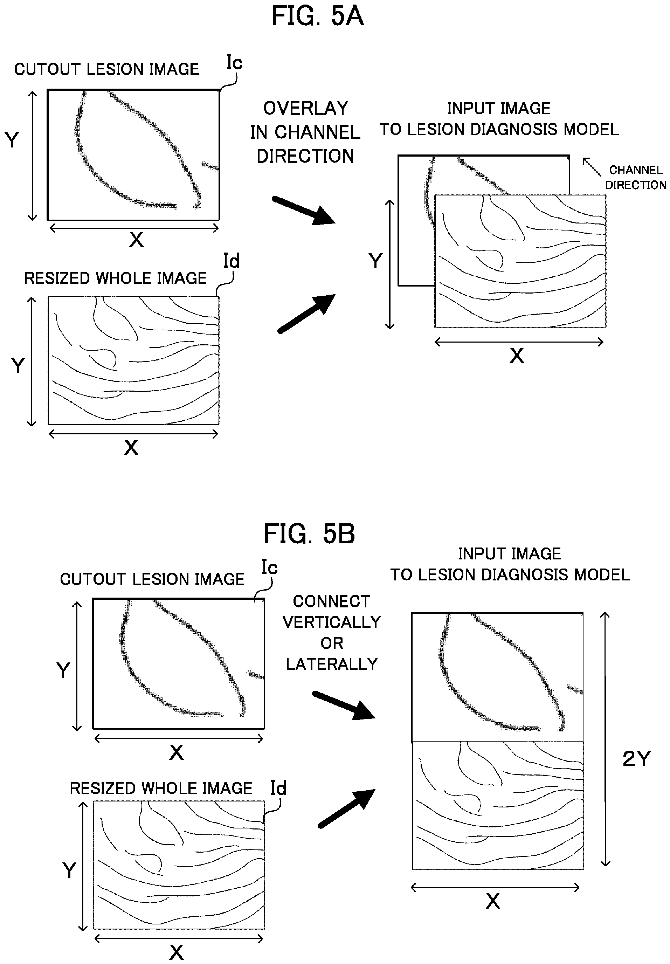

Next, a supplementary description will be given of the generation process of the input image to be inputted to the lesion diagnosis model. FIG. 5A shows a first example of generating an input image to be inputted to the lesion diagnosis model, and FIG. 5B shows a second example of generating the input image to be inputted to the lesion diagnosis model.

In the first example, the lesion diagnosis unit 32 generates an input image to be inputted to the lesion diagnosis model by overlaying the cutout lesion image Ic on the whole size image Id in the channel direction, after adjusting the vertical and horizontal size of the cutout lesion image Ic and the whole size image Id to conform to the input format (here, the horizontal size "X", vertical size "Y", and "Z" channels for each image) of the lesion diagnosis model. In this case, the generated input image is an image with the horizontal size "X", vertical size "Y", and "Z + Z" channels. For example, when the captured image Ia is an RGB image (i.e., Z = 3), the lesion diagnosis unit 32 generates an image with six channels (i.e., Z = 6) in which the cutout lesion image Ic is overlaid on the resized whole image Id in the channel direction as an input image to be inputted to the lesion diagnosis model.

In the second example, the lesion diagnosis unit 32 generates an input image to be inputted to the lesion diagnosis model by combining the cutout lesion image Ic and the resized whole image Id in the vertical or lateral direction. In the example of FIG. 5B, the lesion diagnosis unit 32 generates an input image to be inputted to the lesion diagnosis model by connecting the cutout lesion image Ic and the resized whole image Id (here, the horizontal size "X", vertical size "Y", and "Z" channels for each image) in the vertical direction. In this case, the generated input image is an image with the horizontal size "X", the vertical size "Y + Y", and the "Z" channels. Incidentally, the cutout lesion image Ic and the resized whole image Id are adjusted so that at least the horizontal size is the same when connected in the vertical direction, and is adjusted so that at least the vertical size is the same when connected in the lateral (horizontal) direction.

Then, by generating an input image according to the first example or the second example, the lesion diagnosis unit 32 can identify the classification of the lesion comprehensively considering both the lesion part (including its periphery) and the entire image. Thus, the image processing device 1 can make an accurate diagnosis even for the lesion part which is difficult to diagnose without considering the difference in the surface pattern of the normal area and the lesion part.

The generation of the input image according to the first example or the second example is not an essential process to be executed by the lesion diagnosis unit 32, and another processing block may perform the generation. For example, when another processing block performs the process of overlaying the cutout lesion image Ic on the resized whole image Id in the channel direction, the lesion diagnosis unit 32 acquires an image that is the cutout lesion image Ic overlayed on the resized whole image Id in the channel direction from the other processing block as an input image to be inputted to the lesion diagnosis model. In contrast, when another processing block performs the process of combining the cutout lesion image Ic and the resized whole image Id in the vertical or lateral direction, the lesion diagnosis unit 32 acquires an image, into which the cutout lesion image Ic and the resized whole image Id are combined in the vertical or lateral direction, from the other processing block as an input image to be inputted to the lesion diagnosis model.

Next, a description will be given of the display control of the display device 2 to be executed by the display control unit 33.

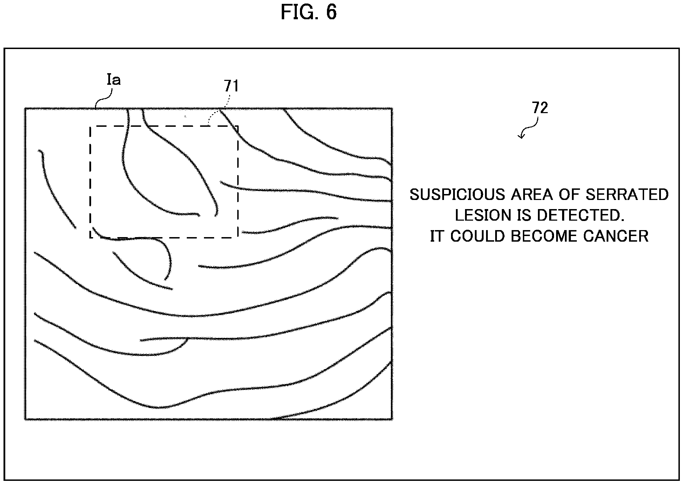

FIG. 6 shows a display example of a display view displayed on the display device 2 in endoscopic inspection. The display control unit 33 of the image processing device 1 causes the display device 2 to display the display view shown in FIG. 6 by transmitting the display information Ib generated based on: the captured image Ia acquired by the captured image acquisition unit 30; the detection result by the lesion part detection unit 31; and the diagnosis result by the lesion diagnosis unit 32.

In the example of FIG. 6, the display control unit 33 of the image processing device 1 displays the latest captured image Ia (i.e., a moving image with a predetermined drawing rate) acquired by the captured image acquisition unit 30 on the display view. Furthermore, on the basis of the diagnosis result supplied from the lesion diagnosis unit 32, the display control unit 33 displays a comment 72 indicates that the lesion part to be classified as a serrated lesion is detected and that the serrated lesion has a possibility of the cancerization. Thus, comment 72 corresponds to information relating to classification and risk of the lesion part. The display control unit 33 may display a comment relating to either the classification or risk of the lesion part, in place of the comment 72.

Furthermore, together with the comment 72 described above, on the basis of the detection result supplied from the lesion part detection unit 31, the display control unit 33 displays, on the captured image Ia, a rectangular frame 71 for highlighting a rectangular area corresponding to the cutout lesion image Ic.

Further, if the diagnosis result supplied from the lesion diagnosis unit 32 indicates that a specific type of lesion (e.g., malignant lesion), the display control unit 33 may output information to notify the inspector of the presence of the specific type of the lesion. In this case, for example, the display control unit 33 may highlight the captured image Ia to be displayed on the display view by the edging effect, or may output a warning sound or guidance sound or the like by the audio output unit 16.

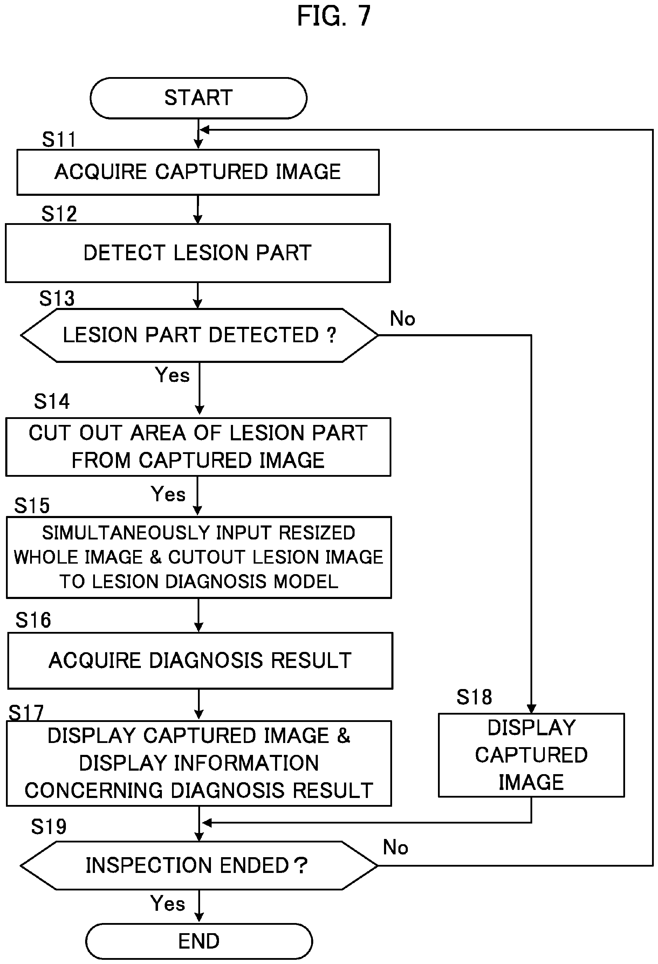

In this way, if the lesion part is detected, the display control unit 33 displays the diagnosis result while specifying the lesion part together with the latest captured image Ia. Thereby, it is possible to suitably support the inspector to grasp the lesion part. The output appearance of the diagnosis result or the like by the display control unit 33 is not limited to the example shown in FIG.7 and it may be various appearances. For example, the display control unit 33, instead of displaying the comment 72, may output a voice corresponding to the comment 72 by the audio output unit 16.

FIG. 7 is an example of a flowchart showing an outline of the process performed by the image processing device 1 when the endoscopic inspection is started according to the first example embodiment.

First, the captured image acquisition unit 30 of the image processing device 1 acquires the captured image Ia (step S11). In this case, the captured image acquisition unit 30 of the image processing device 1 receives the captured image Ia from the endoscope 3 via the interface 13.

Next, the lesion part detection unit 31 of the image processing device 1 performs detection of the lesion part in the captured image Ia (step S12). In this case, the lesion part detection unit 31 acquires the detection result of the lesion part in the captured image Ia from the lesion detection model by inputting the captured image Ia to the lesion detection model configured based on the lesion detection model information D1.

Then, the lesion part detection unit 31 determines whether or not the lesion part is detected (step S13). Then, when not detecting the lesion part (step S13; No), the lesion part detection unit 31 displays the captured image Ia acquired at step S11 on the display device 2 (step S18).

On the other hand, when detecting the lesion part (step S13; Yes), the lesion part detection unit 31 cuts out an area including the detected lesion part from the captured image Ia (step S14). Thereby, the lesion part detection unit 31 generates the cutout lesion image Ic.

Then, the lesion diagnosis unit 32 simultaneously inputs the resized whole image Id, which is acquired by resizing the captured image Ia acquired at step S11, and the cutout lesion image Ic acquired by to the lesion diagnosis model (step S15). In this case, the lesion diagnosis unit 32 inputs, for example, an input image generated according to either the example shown in FIG. 5A or the example shown in FIG. 5B into the lesion diagnosis model configured by the lesion diagnosis model information D2. Then, the lesion diagnosis unit 32 acquires the diagnosis result based on the output of the lesion diagnosis model (step S16). Then, the display control unit 33 causes the display device 2 to display information relating to the diagnosis result acquired at step S16 and the latest captured image Ia (step S17).

After step S17 or step S18, the image processing device 1 determines whether or not the endoscopic inspection has ended (step S19). For example, the image processing device 1 determines that the endoscopic inspection has ended when a predetermined input or the like to the input unit 14 or the operation unit 36 is detected. Then, if it is determined that the endoscopic inspection has ended (step S19; Yes), the image processing device 1 terminates the process of the flowchart. On the other hand, if it is determined that the endoscopic inspection has not ended (step S19; No), the image processing device 1 returns the process to step S11. Then, the image processing device 1 executes the process at step S11 to step S19 for the captured image Ia newly generated by the endoscope 3.

### (4) Modification

Next, modifications suitable for the above-described example embodiment will be described. The following modifications may be applied in combination to the example embodiments described above.

### (First Modification)

The image processing device 1 may process a moving image, which is configured by the captured images Ia captured during the endoscopic inspection, after the endoscopic inspection.

For example, at an arbitrary timing after the inspection, the image processing device 1 sequentially performs processing of the flowchart of FIG. 7 with respect to each of the captured images Ia in the time series constituting the moving image if the moving image to be processed is specified based on the user input or the like by the input unit 14. When determining at step S19 that process of the moving image has ended, the image processing device 1 terminates the processing of the flowchart, and when determining that the process of the moving image has not ended, the image processing device 1 returns the process to step S11 and performs process of the flowchart for the next captured image Ia in the time series.

### (Second Modification)

The lesion detection model information D1 and the lesion diagnosis model information D2 may be stored in a storage device other than the image processing device 1.

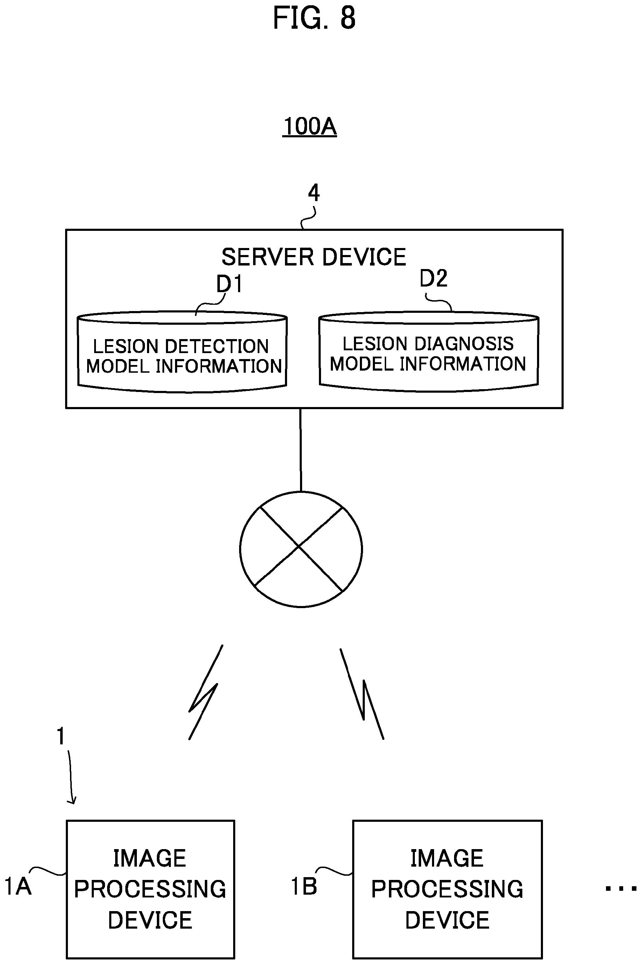

FIG. 8 is a schematic configuration diagram of an endoscopic inspection system 100A according to the second modification. In FIG. 8, for simplicity, the display device 2 and the endoscope 3 and the like are not shown. The endoscopic inspection system 100A shown in FIG. 8 includes a server device 4 that stores the lesion detection model information D1 and the lesion diagnosis model information D2. The endoscopic inspection system 100A also includes a plurality of image processing devices 1 (1A, 1B, ...) capable of data communication with the server device 4 via a network.

In this case, each of the image processing devices 1 makes a reference to the lesion detection model information D1 and the lesion diagnosis model information D2 through the network. In this case, the interface 13 of each image processing device 1 includes a communication interface such as a network adapter for performing communication. In this configuration, as in the above-described example embodiment, each of the image processing devices 1 can suitably perform the detection and diagnosis processing and the display processing regarding the lesion part with reference to the lesion detection model information D1 and the lesion diagnosis model information D2.

### <Second Example Embodiment>

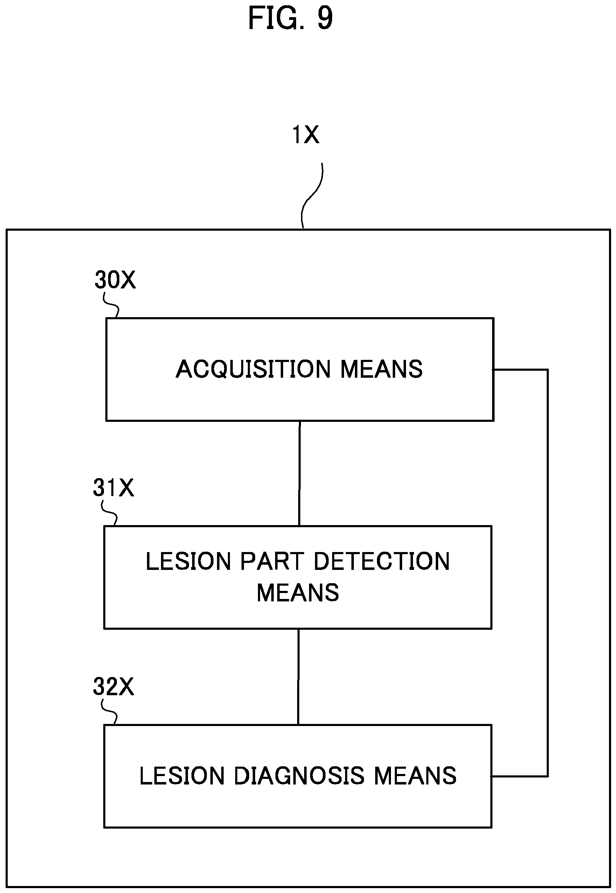

FIG. 9 is a block diagram of the image processing device 1X according to the second example embodiment. The image processing device 1X mainly includes an acquisition means 30X, a lesion part detection means 31X, and a lesion diagnosis means 32X. The image processing device 1X may be configured by a plurality of devices.

The acquisition means 30X acquires a captured image acquired by photographing an inspection target by a photographing unit provided in an endoscope. The acquisition means 30X may be a captured image acquisition unit 30 in the first example embodiment (including a modification, the same is applied hereinafter). The acquisition means 30X may immediately acquire the captured image generated by the photographing unit, or may acquire, at a predetermined timing, the captured image which is generated by photographing unit and which is stored in the storage device in advance.

The lesion part detection means 31X detects a lesion part, in which a lesion formation is suspected, in the captured image. The lesion part detection means 31X may be the lesion part detection unit 31 in the first example embodiment.

The lesion diagnosis means 32X makes a diagnosis relating to the lesion part based on the captured image and a cutout image which indicates the lesion part cut out from the captured image. The lesion diagnosis means 32X may be a lesion diagnosis unit 32 in the first example embodiment.

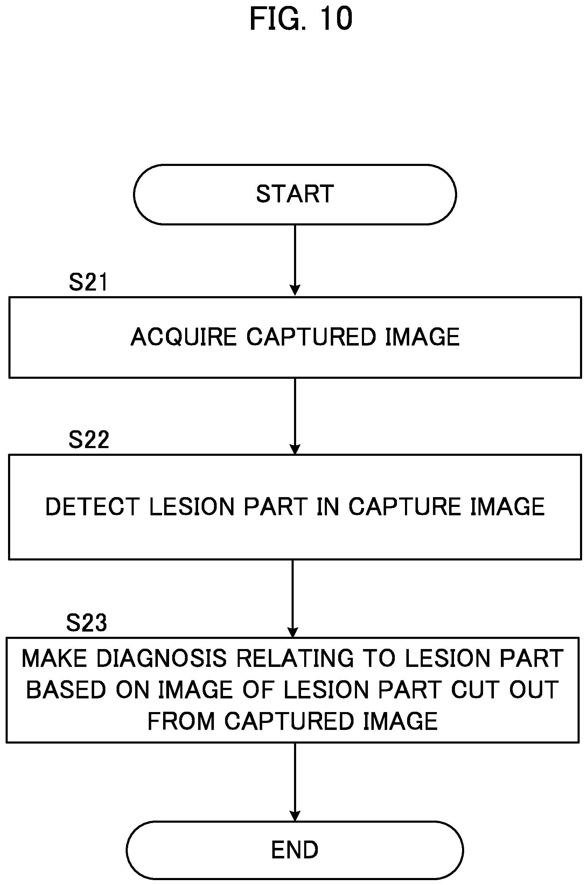

FIG. 10 is an example of a flowchart showing a processing procedure in the second example embodiment. First, the acquisition means 30X acquires a captured image acquired by photographing an inspection target by a photographing unit provided in an endoscope (step S21). Next, the lesion part detection means 31X detects a lesion part, in which a lesion formation is suspected, in the captured image (step S22). Then, the lesion diagnosis means 32X makes a diagnosis relating to the lesion part based on the captured image and a cutout image which indicates the lesion part cut out from the captured image (step S23).

According to the second example embodiment, on the basis of the captured image globally indicating the inspection target and the image that indicates a suspected part of the lesion cut out from the captured image, the image processing device 1X can accurately make the diagnosis of such a lesion that is difficult to diagnose without considering the difference between the surface pattern of the normal aera and the lesion part.

The whole or a part of the example embodiments described above (including modifications, the same applies hereinafter) can be described as, but not limited to, the following Supplementary Notes.

### [Supplementary Note 1]

An image processing device, comprising:
an acquisition means for acquiring a captured image acquired by photographing an inspection target by a photographing unit provided in an endoscope;
a lesion part detection means for detecting a lesion part, in which a lesion formation is suspected, in the captured image; and
a lesion diagnosis means for making a diagnosis relating to the lesion part based on the captured image and a cutout image which indicates the lesion part cut out from the captured image.

### [Supplementary Note 2]

The image processing device according to Supplementary Note 1,
wherein the diagnosis means inputs an image, into which the captured image and the cutout image are combined, to a diagnosis model, and make the diagnosis based on a diagnosis result outputted by the diagnosis model, the diagnosis model being configured to output a diagnosis result relating to a lesion part in an input image when the input image is inputted to the diagnosis model.

### [Supplementary Note 3]

The image processing device according to Supplementary Note 2,
wherein the diagnosis means inputs an image, in which the captured image is overlaid on the cutout image in a channel direction, to the diagnosis model, and makes the diagnosis based on the diagnosis result outputted by the diagnosis model.

### [Supplementary Note 4]

The image processing device according to Supplementary Note 2,
wherein the diagnosis means inputs an image, in which the captured image and the cutout image are connected in a vertical direction or a lateral direction of the images, to the diagnosis model, and makes the diagnosis based on the diagnosis result outputted by the diagnosis model.

### [Supplementary Note 5]

The image processing device according to any one of Supplementary Notes 2 to 4,
wherein the diagnosis means inputs an image, into which the captured image resized based on an input format of the diagnosis model and the cut image are combined, to the diagnosis model, and makes the diagnosis based on the diagnosis result outputted by the diagnosis model.

### [Supplementary Note 6]

The image processing device according to any one of Supplementary Notes 1 to 5,
wherein the diagnosing means makes the diagnosis relating to at least one of a serrated lesion, a hyperplastic polyp, an adenoma, or a carcinoma in situ.

### [Supplementary Note 7]

The image processing device according to any one of Supplementary Notes 1 to 6,
wherein the inspection target is either a large bowel or a stomach.

### [Supplementary Note 8]

The image processing device according to any one of Supplementary Notes 1 to 7, further comprising
an output control means for outputting information based on the diagnosis by a display device or an audio output device.

### [Supplementary Note 9]

The image processing device according to Supplementary Note 8,
wherein the output control means, when detecting the presence of a specific type of a lesion by the diagnosis, causes the display device or the audio output device to output information notifying at least the presence of the lesion as the information based on the diagnosis.

### [Supplementary Note 10]

The image processing device according to Supplementary Note 8 or 9,
wherein the output control means causes the display device or the audio output device to output information relating to at least one of a classification or risk of the lesion part based on the diagnosis as the information based on the diagnosis.

### [Supplementary Note 11]

An image processing method executed by a computer, the image processing method comprising:
acquiring a captured image acquired by photographing an inspection target by a photographing unit provided in an endoscope;
detecting a lesion part, in which a lesion formation is suspected, in the captured image; and
making a diagnosis relating to the lesion part based on the captured image and a cutout image which indicates the lesion part cut out from the captured image.

### [Supplementary Note 12]

A storage medium storing a program executed by a computer, the program causing the computer to:
acquire a captured image acquired by photographing an inspection target by a photographing unit provided in an endoscope;
detect a lesion part, in which a lesion formation is suspected, in the captured image; and
make a diagnosis relating to the lesion part based on the captured image and a cutout image which indicates the lesion part cut out from the captured image.

While the invention has been particularly shown and described with reference to example embodiments thereof, the invention is not limited to these example embodiments. It will be understood by those of ordinary skilled in the art that various changes in form and details may be made therein without departing from the spirit and scope of the present invention as defined by the claims. In other words, it is needless to say that the present invention includes various modifications that could be made by a person skilled in the art according to the entire disclosure including the scope of the claims, and the technical philosophy. All Patent and Non-Patent Literatures mentioned in this specification are incorporated by reference in its entirety.

### DESCRIPTION OF REFERENCE NUMERALS

- 1, 1X: Image processing device
- 2: Display device
- 3: Endoscope
- 4: Server device
- 11: Processor
- 12: Memory
- 13: Interface
- 14: Input unit
- 15: Light source unit
- 16: Audio output unit
- 100, 100A: Endoscopic inspection system

## Claims

1. An image processing device, comprising:
an acquisition means for acquiring a captured image acquired by photographing an inspection target by a photographing unit provided in an endoscope;
a lesion part detection means for detecting a lesion part, in which a lesion formation is suspected, in the captured image; and
a lesion diagnosis means for making a diagnosis relating to the lesion part based on the captured image and a cutout image which indicates the lesion part cut out from the captured image.

2. The image processing device according to claim 1,
wherein the diagnosis means inputs an image, into which the captured image and the cutout image are combined, to a diagnosis model, and make the diagnosis based on a diagnosis result outputted by the diagnosis model, the diagnosis model being configured to output a diagnosis result relating to a lesion part in an input image when the input image is inputted to the diagnosis model.

3. The image processing device according to claim 2,
wherein the diagnosis means inputs an image, in which the captured image is overlaid on the cutout image in a channel direction, to the diagnosis model, and makes the diagnosis based on the diagnosis result outputted by the diagnosis model.

4. The image processing device according to claim 2,
wherein the diagnosis means inputs an image, in which the captured image and the cutout image are connected in a vertical direction or a lateral direction of the images, to the diagnosis model, and makes the diagnosis based on the diagnosis result outputted by the diagnosis model.

5. The image processing device according to any one of claims 2 to 4,
wherein the diagnosis means inputs an image, into which the captured image resized based on an input format of the diagnosis model and the cut image are combined, to the diagnosis model, and makes the diagnosis based on the diagnosis result outputted by the diagnosis model.

6. The image processing device according to any one of claims 1 to 5,
wherein the diagnosing means makes the diagnosis relating to at least one of a serrated lesion, a hyperplastic polyp, an adenoma, or a carcinoma in situ.

7. The image processing device according to any one of claims 1 to 6,
wherein the inspection target is either a large bowel or a stomach.

8. The image processing device according to any one of claims 1 to 7, further comprising
an output control means for outputting information based on the diagnosis by a display device or an audio output device.

9. The image processing device according to claim 8,
wherein the output control means, when detecting the presence of a specific type of a lesion by the diagnosis, causes the display device or the audio output device to output information notifying at least the presence of the lesion as the information based on the diagnosis.

10. The image processing device according to claim 8 or 9,
wherein the output control means causes the display device or the audio output device to output information relating to at least one of a classification or risk of the lesion part based on the diagnosis as the information based on the diagnosis.

11. An image processing method executed by a computer, the image processing method comprising:
acquiring a captured image acquired by photographing an inspection target by a photographing unit provided in an endoscope;
detecting a lesion part, in which a lesion formation is suspected, in the captured image; and
making a diagnosis relating to the lesion part based on the captured image and a cutout image which indicates the lesion part cut out from the captured image.

12. A storage medium storing a program executed by a computer, the program causing the computer to:
acquire a captured image acquired by photographing an inspection target by a photographing unit provided in an endoscope;
detect a lesion part, in which a lesion formation is suspected, in the captured image; and
make a diagnosis relating to the lesion part based on the captured image and a cutout image which indicates the lesion part cut out from the captured image.
